# EUROPÄISCHE PATENTSCHRIFT

(11) **EP 3 534 849 B1**
(45) Veröffentlichungstag und Bekanntmachung des Hinweises auf die Patenterteilung: **23.12.2020**
(21) Anmeldenummer: 17838033.3
(22) Anmeldetag: 26.10.2017
(51) Int. Cl.: A61F 5/01, B25J 9/00, A61B 90/60

(54) **VORRICHTUNG ZUM UNTERSTÜTZEN EINES ARMES**
DEVICE FOR SUPPORTING AN ARM
DISPOSITIF DE SUPPORT D'UN BRAS

(30) Priorität: 07.11.2016 DE 102016121202
(43) Veröffentlichungstag der Anmeldung: 11.09.2019
(73) Patentinhaber: Ottobock SE & Co. KGaA, 37115 Duderstadt (DE)
(72) Erfinder: MIZERA, Oliver, 34134 Kassel (DE); BORNMANN, Jonas, 37115 Duderstadt (DE); BERTELS, Thomas, 37115 Duderstadt (DE); BRÜNJES, Lukas, 37075 Göttingen (DE)
(74) Vertreter: Gramm, Lins & Partner Patent- und Rechtsanwälte PartGmbB
(86) Internationale Anmeldenummer: PCT/EP2017/077492
(87) Internationale Veröffentlichungsnummer: WO 2018/083009

(56) Entgegenhaltungen:
- EP-A1- 1 813 219
- EP-A1- 2 380 529
- WO-A2-2012/099995
- DE-T2- 60 122 483
- US-A1- 2014 158 839

## Beschreibung

Die Erfindung betrifft eine Vorrichtung zum Unterstützen wenigstens eines Armes eines Benutzers, wobei die Vorrichtung wenigstens einen Grundkörper zum Anordnen an einem Rumpf des Benutzers und wenigstens eine Stützeinrichtung zum Stützen des Armes aufweist, wobei die Stützeinrichtung über wenigstens ein Gelenk relativ zu dem Grundkörper bewegbar an dem Grundkörper angeordnet ist.

Durch eine derartige Vorrichtung wird der Arm des Trägers der Vorrichtung gestützt und/oder geführt, was insbesondere für Personen von Vorteil ist, die über Kopf oder mit ausgestreckten und/oder erhobenen Armen arbeiten müssen. Um hier Entlastung zu schaffen, damit diese Personen nicht aus reiner Muskelkraft über eine lange Zeitspanne der wirkenden Gewichtskraft entgegenwirken müssen, sind seit einiger Zeit orthopädietechnische Vorrichtungen bekannt, durch die wenigstens ein Arm entlastet und gestützt werden soll. Eine derartige Vorrichtung kann auch als orthopädietechnische Vorrichtung ausgebildet sein.

So ist beispielsweise aus der WO 2012/099995 A2 eine derartige Vorrichtung bekannt, die über eine Armablagestütze und entsprechende Kompensationselemente verfügt, die beispielsweise gespannte Federn aufweisen können und dadurch permanent eine Kraft auf die Oberarme ausüben, die diese nach oben drückt. Dadurch wird das Arbeiten in dieser ansonsten sehr unbequemen Position erleichtert. Die von den Federelementen aufgebrachte Kraft dient dazu, dass Eigengewicht der Arme zu kompensieren. Durch eine Veränderung der Spannung der verwendeten Federn lässt sich die aufgebrachte Kraft vor der Benutzung der orthopädietechnische Einrichtung einstellen, um beispielsweise den Bedürfnissen unterschiedlicher Träger Rechnung tragen zu können.

Aus der WO 2014/195373 A1 ist eine Vorrichtung bekannt, die zur Unterstützung eines Hebens und Tragens von Lasten durch den Träger der Vorrichtung vorgesehen ist. Sie verfügt über passive Kraftaufbringelemente, wie beispielsweise Blattfedern und/oder über beispielsweise durch einen Motor angetriebene Aktuatoren, die beispielsweise verwendet werden können, um bestimmte Stützelemente aneinander festzulegen und eine gelenkige Bewegung, die ansonsten möglich ist, zu unterbinden.

Aus der nicht vorveröffentlichten DE 10 2016 104 880 ist eine unterstützende Vorrichtung bekannt, bei der die Kraft, die durch die Vorrichtung auf den Arm des Trägers ausgeübt wird, in Abhängigkeit einer Position des Kopfes des Trägers der Vorrichtung eingestellt wird.

Bei den meisten aus dem Stand der Technik bekannten Vorrichtungen wird die unterstützende Wirkung dadurch hervorgerufen, dass permanent eine Kraft auf den zu stützenden Arm ausgeübt wird. Dies bedeutet einerseits, dass zum Bewegen des Armes in die jeweils entgegengesetzte Richtung diese Kraft überwunden werden muss und andererseits, dass auch im angehobenen Zustand der Arme weiterhin die Kraft auf den Arm ausgeübt wird, selbst für den Fall, dass der Träger der Vorrichtung die Position der Arme nicht weiter verändern möchte. Dies kann als störend und unbequem empfunden werden.

Der Erfindung liegt daher die Aufgabe zugrunde, die Nachteile aus dem Stand der Technik zu beheben oder zumindest abzumildern.

Die Erfindung löst die gestellte Aufgabe durch eine Vorrichtung gemäß dem Oberbegriff des Anspruchs 1, die sich dadurch auszeichnet, dass das Gelenk in jeder Position innerhalb eines vorbestimmten Positionsbereiches der Stützeinrichtung relativ zu dem Grundkörper eine Bewegung der Stützeinrichtung relativ zu dem Grundkörper in einer ersten Richtung erlaubt und in einer entgegengesetzten zweiten Richtung blockiert.

Anders als insbesondere in der WO 2012/099995 A2 wird folglich keine permanente Kraft auf den Arm des Trägers der Vorrichtung ausgeübt, sondern das Gelenk in einer Bewegungsrichtung blockiert. Der Benutzer, der beispielsweise mit erhobenen Armen arbeitet, kann folglich wenigstens einen Arm auf der wenigstens einen Stützeinrichtung ablegen, ohne dass die Stützeinrichtung dadurch bewegt würde, sofern das Gelenk eingerichtet ist, die Bewegung in diese Richtung zu verhindern. Ein weiteres Anheben der Arme ist jedoch problemlos möglich, da das Gelenk eine Bewegung in diese Richtung erlaubt.

Vorteilhafterweise kann das Gelenk derart entsperrt werden, dass es eine Bewegung der Stützeinrichtung relativ zu dem Grundkörper in die erste Richtung und in die zweite Richtung erlaubt. Dies ist von Vorteil, wenn die Vorrichtung beispielsweise abgelegt werden soll oder die Arbeit mit erhobenen und/oder gestreckten Armen beendet ist. In diesem Fall muss es möglich sein, beispielsweise die Arme zu senken und damit das Gelenk in der eigentlich gesperrten zweiten Bewegungsrichtung zu bewegen. Für diesen Fall kann das Gelenk in einem zweiten Betriebsmodus betrieben werden, in dem das Gelenk entsperrt ist und eine Bewegung in beiden Richtungen erlaubt.

In einer bevorzugten Ausgestaltung verfügt das Gelenk über eine Welle und eine Schlingfeder, die mit der Welle zusammenwirkt. Vorteilhafterweise verläuft in der Symmetrieachse der Welle, also der Längserstreckung der Welle, die Rotationsachse, um die das Gelenk eine Drehung ermöglicht. Dabei wird eine Drehung in eine erste Richtung erlaubt, während die Drehung in die zweite, entgegengesetzte Richtung durch das Gelenk unterbunden wird. Dies geschieht auf eine konstruktiv sehr einfache Weise durch eine Schlingfeder, die vorzugsweise außen um die Welle herumgelegt ist. Die Schlingfeder besteht vorteilhafterweise aus mehreren Windungen, die um die Welle herum führen und in Kontakt mit der Welle stehen. Aufgrund der vorhandenen Reibung zwischen dem Material der Schlingfeder und der Außenseite der Welle des Gelenkes wird beim Verschwenken der Stützeinrichtung relativ zum Grundkörper, was eine Bewegung der Welle relativ zur Schlingfeder zur Folge hat, eine Kraft auf die Schlingfeder ausgeübt. Wird die Bewegung in die erste Richtung durchgeführt, hat dies zur Folge, dass sich der Durchmesser der Schlingfeder leicht aufweitet und so eine Bewegung der Welle relativ zur Schlingfeder erlaubt. Eine Verschwenkung der wenigstens einen Stützeinrichtung relativ zum Grundkörper in die entgegengesetzte Richtung hat jedoch zur Folge, dass die über die Reibung auf die Schlingfeder aufgebrachte Kraft zu einer Verringerung des Querschnittes der Schlingfeder führen würde, wodurch eine Klemmwirkung, die von der Schlingfeder auf die Welle ausgeübt wird, verstärkt wird. Je stärker das in diese Richtung aufgebrachte Drehmoment oder die in diese Richtung aufgebrachte Kraft ist, desto stärker ist auch dieser allein durch die Reibung zwischen Welle und Schlingfeder hervorgerufene Bremseffekt. Es handelt sich um eine selbstverstärkende Bremse. Bei dieser Ausgestaltung ist für den Betrieb des Gelenkes in diesem Modus keine elektronische Steuerung oder ein Sensor notwendig.

Durch das Gelenk wird die wenigstens eine Stützeinrichtung mit dem Grundkörper verbunden. Ein mit dem Grundkörper verbundenes Bauteil und ein mit der Stützeinrichtung verbundenes zweites Bauteil bilden folglich das Gelenk, wobei die beiden Bauteile des Gelenkes relativ zueinander bewegt werden, sofern die Stützeinrichtung relativ zum Grundkörper bewegt wird. Die Welle ist dabei drehfest mit dem einen der beiden Bauteile, also entweder dem Grundkörper oder der Stützeinrichtung, verbunden, während die Schlingfeder drehfest mit dem jeweils anderen Bauteil verbunden ist. Vorzugsweise ist die Welle mit der Stützeinrichtung und die Schlingfeder mit dem Grundkörper drehfest verbunden.

Vorzugsweise verfügt die Vorrichtung über einen Aktuator, der eingerichtet ist, die Schlaufe zu weiten und so das Gelenk zu entsperren. Dazu verfügt die Schlingfeder vorteilhafterweise über einen Betätigungsvorsprung, der nach radial außen von der Symmetrieachse der Schlingfeder und der Symmetrieachse der Welle, die sich in der Schlingfeder befindet, hervorsteht. Wird nun ein Druck in bezüglich dieser Achse tangentialer Richtung auf diesen Vorsprung ausgeübt, führt dies zu einer Aufweitung des Durchmessers der Schlingfeder gerade so weit, dass die selbstverstärkende Bremswirkung aufgehoben wird, so dass in diesem Zustand das Gelenk eine Bewegung der Welle relativ zur Schlingfeder und damit auch eine Bewegung der wenigstens einen Stützeinrichtung relativ zum Grundkörper in beide Richtungen erlaubt. Der Aktuator kann beispielweise ein Stift, Bolzen oder Stab sein, der beispielsweise longitudinal bewegt wird und so den benötigten Druck auf den Vorsprung der Schlingfeder ausübt.

Alternativ oder zusätzlich dazu verfügt das Gelenk über eine magneto-rheologische Flüssigkeit und wenigstens eine Magnetfelderzeugungseinrichtung, beispielsweise einen Elektromagneten, der so angeordnet ist, dass ein durch den Elektromagneten erzeugtes Magnetfeld zumindest auch auf einen Teil der magneto-rheologischen Flüssigkeit wirkt. Die Magnetfelderzeugungseinrichtung weist insbesondere eine Spule aus einem elektrisch leitfähigen Material auf, deren elektromagnetische Eigenschaften sich durch die Beaufschlagung mit einem elektrischen Strom verändern.

Magneto-rheologische Flüssigkeiten sind Flüssigkeiten, deren Viskosität sich unter Einwirkung eines Magnetfeldes ändert. Herkömmlicherweise nimmt die Viskosität der magneto-rheologischen Flüssigkeit mit steigendem Magnetfeld zu, so dass die Flüssigkeit weniger fließfähig wird. Durch die wenigstens eine Magnetfelderzeugungseinrichtung lässt sich ein steuerbares Magnetfeld erzeugen, so dass auch die Viskosität der magneto-rheologischen Flüssigkeit einstellbar ist. Auf diese Weise lässt sich beispielsweise ein Gelenk konstruieren, bei dem eine Drehmomentübertragung beeinflussbar ist. So kann beispielsweise durch das durch Zu- oder Abschalten eines Magnetfeldes gesperrt oder entsperrt werden. Eine Ausführungsform eines derartigen Gelenks wird beispielsweise in der DE 10 2010 055833 A1 beschrieben

Vorteilhafterweise verfügt die Vorrichtung zumindest über einen Richtungssensor zum Bestimmen der Richtung, in die die Stützeinrichtung relativ zu dem Grundkörper bewegt werden soll, und eine elektrische Steuerung, die eingerichtet ist, die Magnetfelderzeugungseinrichtung in Abhängigkeit von Sensordaten des wenigstens einen Richtungssensors anzusteuern und so das erzeugte Magnetfeld zu beeinflussen und auf die benötige Stärke und gegebenenfalls Richtung einzustellen. Zur Bestimmung der Bewegung der Stützeinrichtung relativ zu dem Grundkörper kann beispielsweise wenigstens ein Momenten- oder/oder wenigstens ein Biegesensor verwendet werden. Alternativ oder zusätzlich dazu kann auch wenigstens ein Beschleunigungs-, Neige- und/oder Inertialsensor verwendet werden. Über wenigstens einen Kraft- und/oder Drucksensor wird vorzugsweise eine auf die Stützeinrichtung und insbesondere auf eine Armschale der Stützeinrichtung aufgebrachte Kraft gemessen. Durch diese und/oder andere Sensoren lassen sich Rückschlüsse auf eine vom Benutzer der Vorrichtung gewünschte oder bereits ausgeführte Bewegung ziehen, die dann zur Steuerung verwendet werden.

Der Sensor ist folglich in der Lage, die Richtung zu bestimmen, in der die wenigstens eine Stützeinrichtung relativ zum Grundkörper durch den Benutzer der Vorrichtung bewegt werden soll. So kann beispielsweise das Vorzeichen einer Kraft, die vom Benutzer auf die Stützeinrichtung ausgeübt wird, bestimmt werden. Soll die Stützeinrichtung dabei in die erste Richtung relativ zum Grundkörper bewegt werden, in der das Gelenk die Bewegung erlaubt, wird der elektrische Strom, der dem Elektromagneten zugeführt wird, so eingestellt, dass die magneto-rheologische Flüssigkeit eine Viskosität aufweist, die eine Bewegung des Gelenkes erlaubt. Dies kann je nach Konstruktion des Gelenkes ein Anschalten oder ein Abschalten des elektrischen Stromes bedeuten. Erkennt der Sensor jedoch eine Kraft, die eine Bewegung der Stützeinrichtung relativ zum Grundkörper in die zweite Richtung zur Folge hätte, in der die Bewegung unterbunden werden soll, ist die elektrische Steuerung eingerichtet die Magnetfelderzeugungseinrichtung so anzusteuern, dass die Viskosität der magneto-rheologischen Flüssigkeit derart beeinflusst wird, dass das Gelenk gesperrt wird.

Soll das Gelenk vollständig entsperrt werden, um eine Bewegung in beide Richtungen zu ermöglichen, muss lediglich ein Betriebsmodus der elektrischen Steuerung entsprechend angepasst werden.

Vorteilhafterweise ist die elektrische Steuerung eingerichtet, eine Stromstärke des elektrischen Stromes in Abhängigkeit der Sensordaten einzustellen und/oder zu variieren. Dies kann beispielsweise dadurch geschehen, dass nicht nur das Vorzeichen einer auf die Stützeinrichtung durch den Benutzer aufgebrachten Kraft, sondern auch der Betrag dieser Kraft durch den Sensor ermittelt oder bestimmt werden kann. Je nach Stärke der Kraft, die eine Bewegung in die zweite Richtung zur Folge hat, kann die elektrische Steuerung auch die Stärke des elektrischen Stromes, mit dem der Elektromagnet beaufschlagt wird, entsprechend anpassen, um sicher dafür zu sorgen, dass eine Bewegung in diese zweite Richtung unterbunden wird. Legt der Benutzer der Vorrichtung beispielsweise nur seinen Arm auf der Stützeinrichtung ab, hat dies eine auf die Stützeinrichtung wirkende Kraft zur Folge, die ein Absenken der Stützeinrichtung zur Folge hätte. Der Sensor detektiert dies und die elektrische Steuerung steuert den elektrischen Strom für den Elektromagneten so, dass das Gelenk in dieser Richtung gesperrt ist. Dazu ist eine vom Gewicht des Armes abhängige Stromstärke nötig. Hat der Benutzer der Vorrichtung bei diesem Vorgehen jedoch zusätzlich beispielsweise ein Werkzeug in der Hand, ist das auf die Stützeinrichtung wirkende Gewicht und damit die ausgeübte Kraft deutlich größer als dies ohne Werkzeug der Fall wäre. Daher kann es von Vorteil sein, auch den elektrischen Strom, der dem Elektromagneten zugeführt wird, in der Stärke anzuheben.

Allgemein gilt eine Bewegung in der zweiten Richtung als verhindert, wenn die beim herkömmlichen Betrieb der Vorrichtung auftretenden Kräfte nicht ausreichend sind, eine derartige Bewegung zu ermöglichen.

In einer bevorzugten Ausgestaltung der Vorrichtung befindet sich die magneto-rheologische Flüssigkeit in einem Spalt und/oder einem Kanal zwischen zwei relativ zueinander bewegbaren Bauteilen des Gelenkes. Dabei ist eine relativ geringe Schichtdicke, beispielsweise von weniger als 1 mm, bevorzugt weniger als 0,5 mm, ausreichend, um Steuern zu können. Bei der Bewegung des Gelenkes treten naturgemäß Schwerkräfte in der magneto-rheologischen Flüssigkeit auf, wenn sich die beiden gegeneinander verschiebbaren oder rotierbaren Bauteile des Gelenkes relativ zueinander bewegen. Ist die Viskosität der magneto-rheologischen Flüssigkeit klein, also die Fließfähigkeit besonders hoch, wirkt die magneto-rheologische Flüssigkeit quasi als Schmiermittel zwischen den beiden sich gegeneinander bewegenden Bauteile und schränkt die Bewegung nicht oder kaum ein. Ist die Viskosität jedoch besonders hoch, blockiert dies eine Bewegung der beiden Bauteile zueinander, so dass auf diese Weise mit relativ geringerem elektrischen Strom eine sehr effektive und effiziente Sperrung des Gelenkes erreicht werden kann.

Vorteilhafterweise verfügt die Vorrichtung über wenigstens einen Permanentmagneten, dessen Magnetfeld zumindest auch auf einen Teil der magneto-rheologischen Flüssigkeit wirkt. Die Viskosität der magneto-rheologischen Flüssigkeit wird durch das Magnetfeld des Permanentmagneten erhöht, so dass ohne elektrischen Strom das Gelenk gesperrt ist. Wird nun der Elektromagnet mit elektrischem Strom beaufschlagt, ist dies je nach Richtung des dadurch erzeugten Magnetfeldes entweder eine Verstärkung der Sperrung, die beispielsweise dann von Vorteil ist, wenn ein Sensor ein besonders starkes Wirken des Drehmomentes detektiert, oder eine Reduzierung des auf die Flüssigkeit wirkenden Magnetfeldes, wodurch das Gelenk in diese Bewegungsrichtung entsperrt werden kann.

Bei allen hier beschriebenen Ausführungsformen liegt ein weiterer Vorteil darin, dass das Gelenk auch im belasteten Zustand entsperrt werden kann. Dies ist insbesondere bei der Verwendung am Arbeitsplatz von Vorteil, da keine unnatürlichen zusätzlichen Bewegungen vorgenommen werden müssen, um das Gelenk zu entlasten, bevor es entsperrt werden kann.

Um das Gelenk vollständig zu entsperren und in den zweiten Betriebsmodus zu schalten, können unterschiedlichste Möglichkeiten verwirklicht werden. So ist es beispielsweise möglich, einen elektrischen Kontakt, der beispielsweise in einem getragenen Handschuh vorhanden sein kann, beispielsweise durch das Anlegen zweier Finger aneinander zu schließen und so das Signal zu geben, das Gelenk zu entsperren. Dadurch kann beispielsweise der Aktuator, der auf den Vorsprung der Schlingfeder wirkt, betätigt werden. Selbstverständlich sind auch andere Möglichkeiten der Steuerung, beispielsweise durch eine Bewegung eines Körperteils, beispielsweise des Kopfes, oder eine Sprachsteuerung möglich.

Vorteilhafterweise verfügt das Gelenk über wenigstens einen Freilauf und/oder einen Ratschenmechanismus, durch den erreicht wird, dass das Gelenk in der wenigstens einen Richtung frei bewegbar ist. Selbstverständlich können derartige Freiläufe oder Ratschenmechanismen auch mit magneto-rheologischen und/oder elektro-rheologischen Flüssigkeiten kombiniert werden.

Wird ein Permanentmagnet verwendet, um beispielsweise eine magneto-rheologische Flüssigkeit dauerhaft mit einem Magnetfeld zu beaufschlagen, kann zum Steuern des Gelenkes auch der Permanentmagnet bewegt, beispielsweise gedreht werden, so dass sich der auf die magneto-rheologische Flüssigkeit wirkende Teil des von dem Permanentmagneten erzeugten Magnetfeldes ändert und so das Gelenk geschaltet werden kann. Selbstverständlich ist dies auch für elektro-rheologische Flüssigkeiten mit einer E-Felderzeugungseinrichtung denkbar.

Vorteilhafterweise verfügt das Gelenk über eine elektro-rheologische Flüssigkeit und wenigstens eine E-Felderzeugungseinrichtung zum Erzeugen eines elektrischen Feldes, die so angeordnet ist, dass ein durch die E-Felderzeugungseinrichtung erzeugtes elektrisches Feld zumindest auch auf einen Teil der elektro-rheologischen Flüssigkeit wirkt.

Mit Hilfe der beiliegenden Zeichnungen wird anliegend ein Ausführungsbeispiel der vorliegenden Erfindung näher erläutert. Es zeigt:
- Figur 1 -: eine orthopädietechnische Vorrichtung gemäß einem ersten Ausführungsbeispiel der vorliegenden Erfindung im angelegten Zustand,
- Figur 2 -: die schematische Darstellung eines Gelenkes für eine orthopädietechnische Vorrichtung gemäß einem weiteren Ausführungsbeispiel der vorliegenden Erfindung,
- Figur 3 -: das Gelenk aus Figur 2 aus einer anderen Perspektive,
- Figur 4 -: die Darstellung aus Figur 3 mit dem Teil einer Stützeinrichtung,
- Figur 5 -: die Darstellung aus Figur 4 aus einer anderen Perspektive
- Figur 6 -: eine schematische Schnittdarstellung durch ein Gelenk

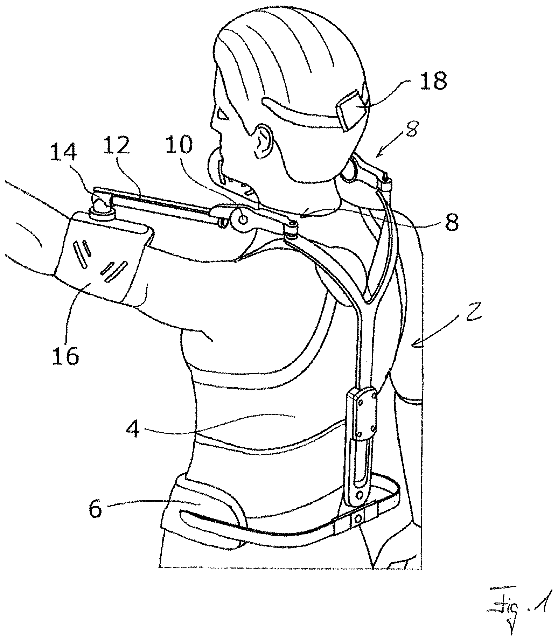

Figur 1 zeigt eine orthopädietechnische Vorrichtung gemäß einem ersten Ausführungsbeispiel der vorliegenden Erfindung im angelegten Zustand. Sie verfügt über einen Grundkörper 2, der in Form von Gurten und Haltestangen ausgebildet und am Rumpf 4 des Trägers der Vorrichtung angeordnet ist. Über einen Beckengurt 6 wird die benötigte Stabilität erreicht. Er dient gleichzeitig als Krafteinleitungselement.

Die Vorrichtung verfügt über zwei Stützeinrichtungen 8, die jeweils ein Gelenk 10 aufweisen, an dem eine Teleskopstange 12 angeordnet ist. An dieser befindet sich ein Schlittenelement 14, an dem sich die Armschale 16 befindet.

Der in Figur 1 links dargestellte Arm ist im erhobenen Zustand dargestellt. Die Teleskopstange 12 ist eingefahren und das Schlittenelement 14 ist somit relativ nah an der Schulter des Trägers positioniert. Ein Absenken des Armes aus dieser Position würde zu einer Verschwenkung im Gelenk 10 führen, die einer Bewegung in die zweite Richtung entspricht. Diese wird durch das Gelenk 10 verhindert, solange es nicht entsperrt ist. Der Träger trägt ein Kopfelement 18, das im gezeigten Ausführungsbeispiel einen Neigungswinkelsensor enthält. Mit diesem ist es möglich, den Neigungswinkel oder die Position des Kopfes des Trägers zu bestimmen. Über die Daten, die dieser Sensor ermittelt, ist eine nicht gezeigte elektrische Steuerung in der Lage, die jeweiligen Gelenke 10 aus dem ersten Betriebsmodus, in dem die Bewegung des Gelenkes nur in die erste Richtung erlaubt und in die zweite Richtung verhindert ist, in den zweiten Betriebsmodus, in dem das Gelenk entsperrt und eine Bewegung in beide Richtung erlaubt ist, zu schalten.

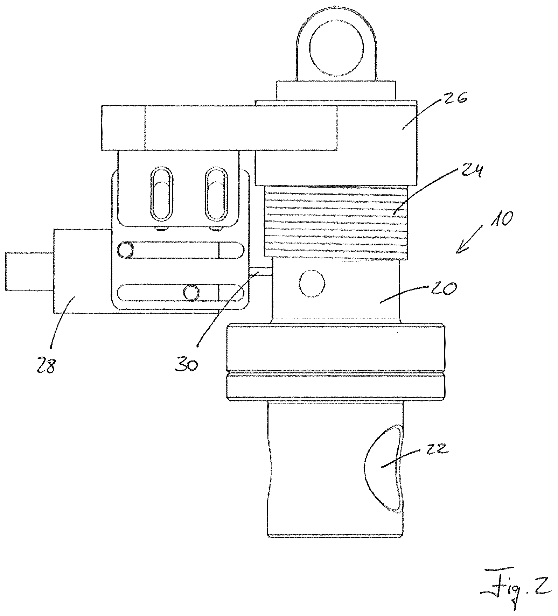

Figur 2 zeigt eine vergrößerte Darstellung des Gelenkes 10. Es verfügt über eine Welle 20, die über eine Bohrung 22 verfügt, in die die Teleskopstange 12 eingeführt werden kann. Die Welle 20 ist somit drehfest mit der Stützeinrichtung 8 verbunden. Das in Figur 2 dargestellte Gelenk 10 verfügt zudem über eine Schlingfeder 24, die sich außen um die Welle 20 in mehreren Windungen erstreckt. Sie ist drehfest mit einem Bauteil 26 verbunden, das die Verbindung zum Grundkörper 2 herstellt.

Wird nun die Welle 20 um ihre Längsachse herumgedreht, wird eine Kraft durch die Reibung zwischen der Schlingfeder 24 und die Welle 20 auf die Schlingfeder 24 ausgeübt, die eine Verkleinerung oder Vergrößerung des Querschnittes, also des Durchmessers der Windungen, der Schlingfeder 24 zur Folge hat. Dadurch wird entweder die Bewegung erlaubt, da sich der Durchmesser der Schlingfeder 24 vergrößert oder das Gelenk gesperrt, da die Bewegung eine Verkleinerung des Durchmessers der Schlingfeder 24 zur Folge hätte.

Das in Figur 2 gezeigte Gelenk 10 verfügt zudem über einen Aktuator 28, der über einen Stift 30 verfügt, der im gezeigten Ausführungsbeispiel nach rechts und links bewegt werden kann.

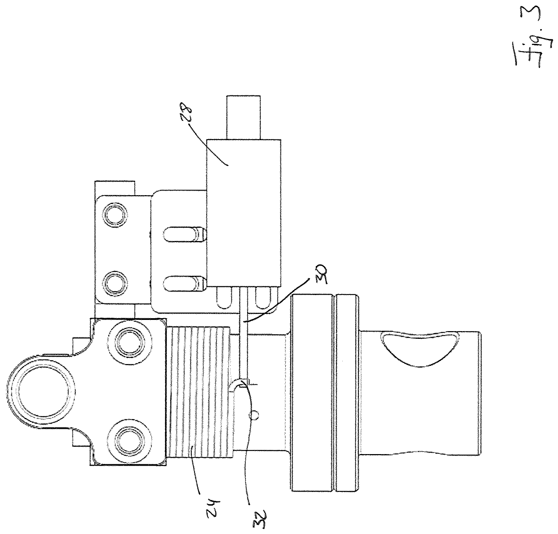

In Figur 3 ist dargestellt, dass der Stift 30 des Aktuators 28 mit einem Vorsprung 32 der Schlingfeder 24 zusammenwirkt. Wird der Stift 30 des Aktuators 28 bewegt, wird der Vorsprung 32 im gezeigten Ausführungsbeispiel nach links bewegt, was eine Vergrößerung des Querschnittes der Schlingfeder 24 und damit eine Freigabe des Gelenkes zur Folge hat.

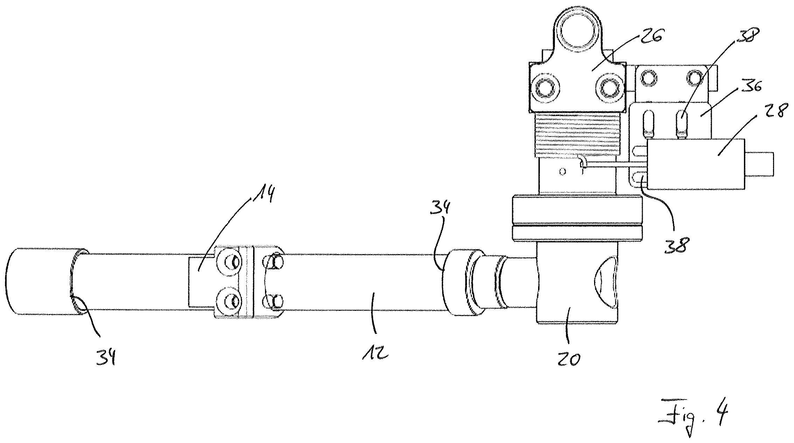

Figur 4 zeigt die Darstellung aus Figur 3 mit einer an der Welle 20 angeordneten Teleskopstange 12. An ihr befindet sich das Schlittenelement 14, das auf der Teleskopstange 12 zwischen zwei Anschlägen 34 verschiebbar gelagert ist.

Der Aktuator 28 ist über eine Befestigungsplatte 36 mit dem Bauteil 26 verbunden. Über Langlöcher 38 lässt sich die Position des Aktuators 28 relativ zum Bauteil 26 individuell so einstellen, dass der Stift 30 mit dem Vorsprung 32 zusammenwirkt.

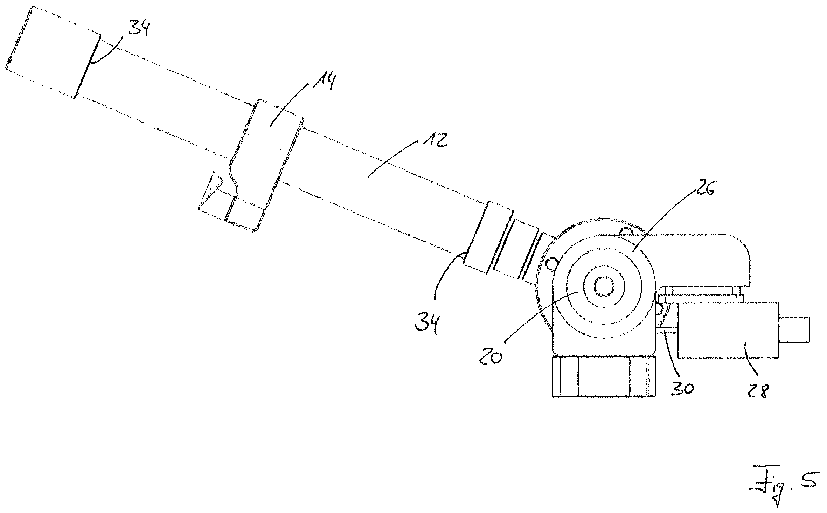

Figur 5 zeigt die Anordnung aus Figur 4 aus einer anderen Perspektive. Man erkennt die Welle 20, die sich im Bauteil 26 drehbar befindet. An der Teleskopstange 12 befindet sich das verschiebbar gelagerte Schlittenelement 14, dessen Bewegung durch die beiden Anschläge 34 begrenzt ist. An ihm kann die in Figur 5 nicht dargestellte Armschale 16 angeordnet werden.

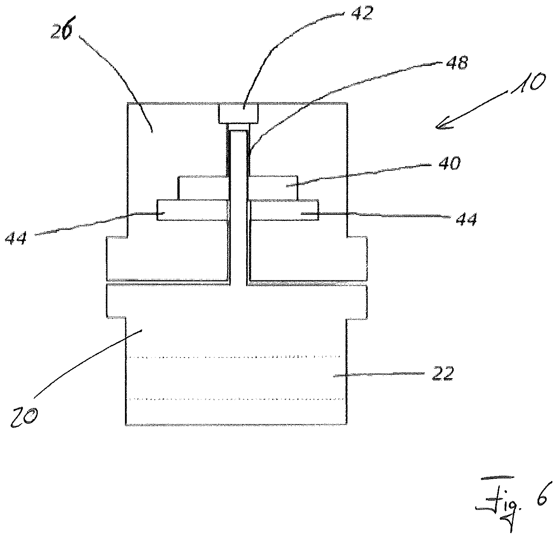

Figur 6 zeigt eine schematische Schnittdarstellung durch das Gelenk 10. Man erkennt die Bohrung 22, die sich an der Welle 20, also dem drehbar gelagerten Bauteil des Gelenkes 10, befindet. Am Bauteil 26 befindet sich eine Vertiefung, in der ein Stift der Welle 20 angeordnet ist. Am Abschluss befindet sich ein Permanentmagnet 42. Im Inneren des Bauteils 26 befindet sich ein Hohlraum, in dem sich neben einer magneto-rheologischen Flüssigkeit 40 auch eine Magnetfelderzeugungseinrichtung 44, die beispielsweise ein Elektromagnet sein kann, befindet. In der gezeigten Ausführungsform ist die magneto-rheologische Flüssigkeit 40 dem Magnetfeld des Permanentmagneten 42 ausgesetzt, so dass sie über eine erhöhte Viskosität und damit eine schlechtere Fließfähigkeit verfügt. Das Gelenk ist folglich gesperrt, da die Welle 20 nicht relativ zum Bauteil 26 bewegt werden kann. Wird jedoch durch die Magnetfelderzeugungseinrichtung 44 ein dem Magnetfeld des Permanentmagneten 42 entgegengesetztes Magnetfeld aufgebaut, heben sich die beiden Magnetfelder in ihrer Wirkung auf und die magneto-rheologische Flüssigkeit erhält eine deutlich verringerte Viskosität und damit eine verbesserte Fließfähigkeit, so dass die beiden Bauteile 20, 26 relativ zueinander bewegt werden können. Die magneto-rheologische Flüssigkeit 40 befindet sich sowohl in dem bereits genannten Hohlraum als auch in einem Spalt 48.

### Bezugszeichenliste

- 2: Grundkörper
- 4: Rumpf
- 6: Beckengurt
- 8: Stützeinrichtung
- 10: Gelenk
- 12: Teleskopstange
- 14: Schlittenelement
- 16: Armschale
- 18: Kopfelement
- 20: Welle
- 22: Bohrung
- 24: Schlingfeder
- 26: Bauteil
- 28: Aktuator
- 30: Stift
- 32: Vorsprung
- 34: Anschlag
- 36: Befestigungsplatte
- 38: Langloch
- 40: magneto-rheologische Einheit
- 42: Permanentmagnet
- 44: Magnetfelderzeugungseinrichtung
- 48: Spalt

## Patentansprüche

1. Vorrichtung zum Unterstützen wenigstens eines Armes eines Benutzers, wobei die Vorrichtung
- wenigstens einen Grundkörper (2) zum Anordnen an einem Rumpf (4) des Benutzers und
- wenigstens eine Stützeinrichtung (8) zum Stützen des Armes aufweist, wobei die Stützeinrichtung (8) über wenigstens ein Gelenk (10) relativ zu dem Grundkörper (2) bewegbar an dem Grundkörper (2) angeordnet ist, wobei das Gelenk (10) in jeder Position innerhalb eines vorbestimmten Positionsbereiches der Stützeinrichtung (8) relativ zu dem Grundkörper (2) eine Bewegung der Stützeinrichtung (8) relativ zu dem Grundkörper (2) in einer ersten Richtung erlaubt, **dadurch gekennzeichnet dass** das Gelenk in einer entgegengesetzten zweiten Richtung blockiert.

2. Vorrichtung nach Anspruch 1, **dadurch gekennzeichnet, dass** das Gelenk (10) derart entsperrt werden kann, dass es eine Bewegung der Stützeinrichtung (8) relativ zu dem Grundkörper (2) in die erste Richtung und in die zweite Richtung erlaubt.

3. Vorrichtung nach Anspruch 1 oder 2, **dadurch gekennzeichnet, dass** das Gelenk (10) eine Welle (20) und eine Schlingfeder (24) aufweist, die mit der Welle (20) zusammenwirkt.

4. Vorrichtung nach Anspruch 3, **dadurch gekennzeichnet, dass** die Vorrichtung einen Aktuator (28) aufweist, der eingerichtet ist, die Schlingfeder (24) aufzuweiten und so das Gelenk (10) zu entsperren.

5. Vorrichtung nach einem der vorstehenden Ansprüche, **dadurch gekennzeichnet, dass** das Gelenk (10) eine magneto-rheologische Flüssigkeit (40) und wenigstens eine Magnetfelderzeugungseinrichtung (44), vorzugsweise wenigstens einen Elektromagneten aufweist, die so angeordnet ist, dass ein durch die Magnetfelderzeugungseinrichtung (44) erzeugtes Magnetfeld zumindest auch auf einen Teil der magneto-rheologischen Flüssigkeit (40) wirkt.

6. Vorrichtung nach Anspruch 5, **dadurch gekennzeichnet, dass** die Vorrichtung wenigstens einen Richtungssensor zum Bestimmen der Richtung, in die die Stützeinrichtung (8) relativ zu dem Grundkörper (2) bewegt werden soll, und eine elektrische Steuerung aufweist, die eingerichtet ist, die Magnetfelderzeugungseinrichtung (44) in Abhängigkeit von Sensordaten des Richtungssensors anzusteuern.

7. Vorrichtung nach Anspruch 6, **dadurch gekennzeichnet, dass** die elektrische Steuerung eingerichtet ist, ein durch die Magnetfelderzeugungseinrichtung (44) erzeugbares Magnetfeld in Abhängigkeit der Sensordaten einzustellen und/oder zu variieren.

8. Vorrichtung nach einem der Ansprüche 5 bis 7, **dadurch gekennzeichnet, dass** sich die magneto-rheologische Flüssigkeit (40) in einem Spalt (48) und/oder einem Kanal zwischen zwei relativ zueinander bewegbaren Bauteilen des Gelenkes (10) befindet.

9. Vorrichtung nach einem der Ansprüche 5 bis 8, **dadurch gekennzeichnet, dass** die Vorrichtung wenigstens einen Permanentmagneten (42) aufweist, dessen Magnetfeld zumindest auch auf einen Teil der magneto-rheologischen Flüssigkeit (40) wirkt.

## Claims

1. A device for supporting at least one arm of a user, wherein the device has
- at least one main body (2) for arranging on a torso (4) of the user and
- at least one supporting device (8) for supporting the arm, wherein the supporting device (8) is arranged on the main body (2) via at least one joint (10) so that it can be moved relative to the main body (2),
wherein, in every position within a predetermined position range of the supporting system (8) relative to the main body (2), the joint (10) allows a movement of the supporting system (8) relative to the main body (2) in a first direction, **characterized in that** the joint (10) is blocked in an opposite second direction.

2. The device according to claim 1, **characterized by the fact that** the joint (10) can be blocked in such a way that it allows a movement of the support system (8) relative to the main body (2) in the first direction and the second direction.

3. The device according to claim 1 or 2, **characterized by the fact that** the joint (10) features a shaft (20) and a wrap spring (24) that interacts with the shaft (20).

4. The device according to claim 3, **characterized by the fact that** the device has an actuator (28), which is configured to expand the wrap spring (24) and thereby unblock the joint (10).

5. The device according to one of the above claims, **characterized by the fact that** the joint (10) comprises a magnetorheological fluid (40) and at least one magnetic field generation device (44), preferably at least one electromagnet, which is arranged in such a way that a magnetic field generated by the electromagnetic field generation device (44) has an effect on at least one part of the magnetorheological fluid (40).

6. The device according to claim 5, **characterized by the fact that** the device features at least one directional sensor for determining the direction in which the supporting system (8) is to be moved relative to the main body (2), and an electric control system that is configured to control the magnetic field generation device (44) depending on sensor data from the directional sensor.

7. The device according to claim 6, **characterized by the fact that** the electric control system is configured to adjust and/or vary a magnetic field, which can be generated by the magnetic field generation device (44), depending on the sensor data.

8. The device according to one of the claims 5 to 7, **characterized by the fact that** the magnetorheological fluid (40) is situated in a gap (48) and/or a channel between two structural components of the joint (10) which can be moved relative to one another.

9. The device according to one of the claims 5 to 8, **characterized by the fact that** the device comprises at least one permanent magnet (42), the magnetic field of which acts on at least on one part of the magnetorheological fluid (40).

## Revendications

1. Dispositif pour soutenir au moins un bras d'un utilisateur, ledit dispositif
- comprend au moins un corps de base (2) destiné à être agencé sur un tronc (4) de l'utilisateur, et
- comprend au moins un dispositif de soutien (8) pour soutenir le bras, ledit dispositif de soutien (8) étant agencé sur le corps de base (2) au moyen d'au moins une articulation (10) de façon déplaçable par rapport au corps de base (2),
dans lequel l'articulation (10) permet, dans toute position à l'intérieur d'une plage de positions prédéterminée du dispositif de soutien (8) par rapport au corps de base (2), un déplacement du dispositif de soutien (8) dans une première direction par rapport au corps de base (2),
**caractérisé en ce que** l'articulation bloque dans une seconde direction opposée.

2. Dispositif selon la revendication 1, **caractérisé en ce que** l'articulation (10) est susceptible d'être débloquée de telle façon qu'elle permet un déplacement du dispositif de soutien (8) dans la première direction et dans la seconde direction par rapport au corps de base (2).

3. Dispositif selon la revendication 1 ou 2, **caractérisé en ce que** l'articulation (10) comprend un axe (20) et un ressort en boucle (24) qui coopère avec l'axe (20).

4. Dispositif selon la revendication 3, **caractérisé en ce que** le dispositif comprend un actionneur (28) qui est conçu pour évaser le ressort en boucle (24) et ainsi pour débloquer l'articulation (10).

5. Dispositif selon l'une des revendications précédentes, **caractérisé en ce que** l'articulation (10) comprend un liquide magnéto-rhéologique (40) et au moins un dispositif de génération de champ magnétique (44), de préférence au moins un électroaimant, qui est agencé de telle façon qu'un champ magnétique engendré par le dispositif de génération de champ magnétique (44) agit sur une partie du liquide magnéto-rhéologique (40).

6. Dispositif selon la revendication 5, **caractérisé en ce que** le dispositif comprend au moins un capteur de direction pour déterminer la direction dans laquelle le dispositif de soutien (5) doit être déplacé par rapport au corps de base (2), et une commande électrique qui est conçue pour piloter le dispositif de génération de champ magnétique (44) en fonction des données provenant du capteur de direction.

7. Dispositif selon la revendication 6, **caractérisé en ce que** la commande électrique est conçue pour établir et/ou pour faire varier un champ magnétique qui peut être engendré par le dispositif de génération de champ magnétique (44) en fonction des données du capteur.

8. Dispositif selon l'une des revendications 5 à 7, **caractérisé en ce que** le liquide magnéto-rhéologique (40) se trouve dans un intervalle (48) et/ou dans un canal entre deux composants, déplaçables l'un par rapport à l'autre, de l'articulation (10).

9. Dispositif selon l'une des revendications 5 à 8, **caractérisé en ce que** le dispositif comprend au moins un aimant permanent (42) dont le champ magnétique agit au moins sur une partie du liquide magnéto-rhéologique (40).
